# EUROPÄISCHE PATENTANMELDUNG

(11) **EP 0 905 042 A1**
(43) Veröffentlichungstag der Anmeldung: **31.03.1999**
(21) Anmeldenummer: 97810677.1
(22) Anmeldetag: 17.09.1997
(51) Int. Cl.: B65D 75/34, A61K 9/20, B29C 51/08

(54) **Blister- oder Durchdrückpackung**

(71) Anmelder: Alusuisse Technology & Management AG, 8212 Neuhausen am Rheinfall (CH)
(72) Erfinder: Oster, Heinz, 8245 Feuerthalen (CH); Zeiter, Patrik, 8280 Bülach (CH); Bossel, Daniel, 8200 Schaffhausen (CH)

(57) **Zusammenfassung**

Blister- oder Durchdrückpackungen mit Bodenteil und Deckel aus metall- und/oder kunststoffhaltigen Folien, mit zumindest in den Bodenteilen eingeformten Vertiefungen, wobei jede Vertiefung eine Seitenwandung und einen Boden aufweist, und die Böden der Vertiefungen eine positive oder negative Prägung aufweisen.

Beispielsweise für eine Lyophilisation oder Gefriertrocknung von pharmazeutischen Produkten werden in die Vertiefungen der Bodenteile der Blister- oder Durchdrückpackungen das pharmazeutische Produkt als Flüssigkeit eingefüllt und unter Vakuum Temperaturen bis zu - 196°C ausgesetzt. Dabei gefrieren und verdampfen oder sublimieren die ursprünglich flüssigen Anteile und es bleiben die Feststoffe in Tablettenform, zurück. Die entstehenden Tabletten weisen das Abbild der Prägungen, die an den Böden der Vertiefungen angebracht worden sind, auf. Somit ist eine Charakterisierung der Tabletten ohne einen Bedruckungs- oder Färbeprozess mittels eines Tablettierverfahrens möglich.

## Beschreibung

Vorliegende Erfindung betrifft Blister- oder Durchdrückpackungen, enthaltend metall- und/oder kunststoffhaltigen Folien mit darin eingeformten Vertiefungen, wobei die Vertiefungen Seitenwandungen und Böden aufweisen, ein Verfahren zu deren Herstellung und deren Verwendung.

Es ist aus der EP-B-0 646 367 bekannt, für die Befüllung von Blisterpackungen ein Bodenteil, mit einer Mehrzahl von Vertiefungen, vorzulegen. Die im Bodenteil aufzunehmenden pharmazeutischen Präparate werden als flüssige Zubereitung portionenweise in jede Vertiefungen des Bodenteils einer Blisterpackung gefüllt. Die flüssige Zubereitung im Bodenteil wird eingefroren und einem Gefriertrocknungprozess unterworfen, dabei sublimiert die gefrorene Flüssigkeit ab und es bleiben die Feststoffe in jeder Vertiefung als Tablette zurück. Anschliessend kann der Bodenteil mit einer Deckelfolie verschlossen werden. Als Werkstoff für den Bodenteil wird ein mehrschichtiger Film beschrieben, aus einer undurchlässigen Zwischenschicht, die beidseitig äussere Schichten eines Materials gleicher thermischer Ausdehnungskoeffizienten aufweist.

Die WO 94/19184 beschreibt einen Verbundwerkstoff aus einer Metallschicht, wie einer Aluminiumfolie, und beidseitig der Metallschicht einer Kunststoffschicht, beispielsweise einer Polyamidschicht. Der Verbundwerkstoff kann zu Bodenteilen für Blisterpackungen tief oder streckgezogen werden. Die Bodenteile finden insbesondere Verwendung für die Aufnahme von flüssigen Portionen von pharmazeutischen Präparaten, die zur Erzeugung einer Tablette einem Gefriertrocknungsprozess unterworfen werden. Die beschriebenen Verbundwerkstotte und die beschriebene Verformung der Verbundwerkstoffe ermöglichen ein zuverlässiges Befüllen und anschliessendes weiteres Verarbeiten der Blisterpackungen. Die Bodenteile zeigen bei der Gefriertrocknung eine hohe Planlage und keine Wölbtendenz. Damit ist sichergestellt, dass die flüssige Zubereitung vor und während des Gefriertrocknens in der jeweiligen Vertiefung bleibt und nicht ausläuft.

Die feste Darreichungsform des Medikamentes in Tablettenform entsteht somit in der Endverpackung. Damit wird jedoch die Kennzeichnung der während des Gefriertrocknens gebildeten Tablette durch einen Aufdruck verunmöglicht. Beispielsweise verlangt die US-Gesundheitsbehörde FDA eine individuelle Kennzeichnung jeder einzelnen Tablette. Die Kennzeichnung soll u.a. eine Differenzierung zwischen Präparaten mit hohem Wirkstoffgehalt und niedrigem Wirkstoffgehalt ermöglichen und die entsprechenden Vergiftungsgefahren bannen.

Aufgabe vorliegender Erfindung ist es, eine Lösung vorzuschlagen, die es ermöglicht, einzelne Tabletten oder jede einzelne Tablette auch heim beschriebenen Herstellungsverfahren zu kennzeichnen.

Erfindungsgemäss wird dies dadurch erreicht, dass wenigstens der Boden einer Vertiefung eine positive oder negative Prägung aufweist.

Als weitere Ausdrücke mit der Bedeutung von Vertiefungen können auch Näpfchen oder Höfe genannt werden. Bevorzugt weisen alle Böden der Vertiefungen eine positive oder negative Prägung auf. Die Prägung ist zumindest auf der gegen die Innenseite einer Packung gerichteten Seite der Böden vorhanden. Die kunststoff- und/oder metallhaltigen Folien mit den daran angebrachten Vertiefungen bilden in der Regel die Bodenteile der Blister- oder Durchdrückpackungen. Die Bodenteile können mit einer Deckelfolie versehen werden oder als Deckel kann ein dem Bodenteil entsprechendes oder ähnliches Gleichteil angewendet werden.

Das Prägung kann beispielsweise ein Schriftbild, ein Brailleschriftbild, ein Logogramm, ein Piktogramm, ein Ideogramm oder ein Relief darstellen. Die Prägung kann der Tablette auch eine originelle kennzeichnende Form oder kennzeichnende Formteile verleihen.

Alle oder einzelne Böden der Vertiefungen können beispielsweise eine positive Prägung auf weisen und das pharmazeutische Produkt, d.h. die Tablette, weist deshalb auf der dem Boden zugewandten Seite -- als Abbild des positiven Prägemusters -- das Negativ der Prägung auf. Es können auch alle oder einzelne Böden eine negative Prägung aufweisen und das pharmazeutische Produkt weist deshalb auf der dem Boden zugewandten Seite -- als Abbild der negativen Prägung -- das Positiv der Prägung auf. Es ist auch möglich an der gleichen Blister- oder Durchdrückpackung neben Böden mit negativen auch solche mit positiven Prägungen vorzusehen.

Die metall- und/oder kunststoffhaltigen Folien zur Herstellung der Blister- oder Durchdrückpackungen können beispielsweise Verbundwerkstoffe, Schichtstoffe oder Laminate sein. Die metall- und/oder kunstoffhaltigen Folien sind vorzugsweise Laminate aus einer undurchlässigen Zwischenschicht aus Metall und beidseitig der Zwischenschicht angeordneten äusseren Schichten aus kunststoffhaltigen Folien. Die undurchlässige Zwischenschicht weist insbesondere eine hohe oder absolute Sperrwirkung gegen den Durchtritt von Dämpfen, wie Wasserdampf, und Gasen, wie Luft, resp. Sauerstoff, und Geruchs- und Aromastoffen und dergl. auf. Die äusseren Schichten weisen bevorzugt gleiche thermische Ausdehnungkoeffizienten auf.

Als Metalle können beispielsweise Eisen, Stahl oder Kupfer und bevorzugt Aluminium und seine Legierungen, als Folie angewendet werden. Zweckmässig ist eine Folie aus Aluminium, wie z.B. AlFeSi oder AlFeSiMn usw., mit einer Reinheit von 98,3 % und höher, bevorzugt von 98,5 % und höher bis höchstens 99,0 %. Weiter zweckmässig sind Aluminiumlegierungen, z.B. des Typs AA 8014, AA 8079, dabei insbesondere AA 8079 mit einer Reinheit von 98,6 %, AA 8101, vorzugsweise AA 8021 und insbesondere AA 8021 mit einer Reinheit von 98,5 %.

Insbesondere bevorzugt ist als Aluminiumfolie ein ununterbrochenes, weichgeglühtes, feinkörniges Aluminiumdünnband, insbesondere mit wenigstens 3 und besonders bevorzugt 5 Kornlagen über die Dicke des Bandes.

Die Oberfläche der Metallschicht und insbesondere der Aluminiumschicht, ist vorzugsweise homogen, ohne Restschmiermittel und mit definierter Oberfläche. Die Aluminiumoberflächen können beispielsweise mit Einbrennlackierungen auf Epoxy- oder Phenolbasis oder mit Konversionsschichten, wie Mischoxid- und/oder Hydratschichten, behandelt sein. Weiter können die Oberflächen durch eine Koronaentladungsbehandlung vorbehandelt sein.

Die Dicke der Metallschicht kann von 8 bis 80 µm, zweckmässig von 40 bis 70 µm und insbesondere von 45 bis 60 µm betragen.

Als Beispiele von kunststofthaltigen Folien sind insbesondere Folien auf Basis von thermoplastischen Kunststoffen zu nennen. Typische Thermoplaste sind Cycloolefin-Copolymere, Polyolefine, Polyvinylchlorid, Polyester und bevorzugt Polyamide.

Typische Beispiele von thermoplastischen Kunststoffen aus der Reihe der Polyolefine sind ungereckte oder uni- oder biaxial gereckte Polyethylene, wie LDPE, LLDPE, LMDPE, MDPE, HDPE usw., Polypropylene, wie cast-Polypropylen und uni- oder biaxial gereckte Polypropylene der Reihe der amorphen, teilamorphen, teilkristallinen, kristallinen oder hochkristallinen Polypropylene, oder aus der Reihe der Polyester, wie Polyalkylenterephthalate oder Polyalkylenisophthalate und insbesondere das Polyethylenterephthalat.

Die Kunststoffschichten enthalten bevorzugt einen Thermoplasten auf Polyamid-Basis oder können überwiegend einen Thermoplasten auf Polyamid-Basis enthalten oder können aus einem Thermoplasten auf Polyamid-Basis bestehen.

Zu den Thermoplasten auf Polyamid-Basis gehören beispielsweise die Polyamide Polyamid 6, ein Homopolymerisat aus ε-Caprolactam (Polycaprolactam); Polyamid 11, Polyamid 12, ein Homopolymerisat aus ω-Laurinlactam (Polylaurinlactam); Polyamid 6,6, ein Homopolykondensat aus Hexamethylendiamin und Adipinsäure (Polyhexamethylenadipamid); Polyamid 6,10, ein Homopolykondensat aus Hexamethylendiamin und Sebacinsäure (Polyhexamethylensebacamid); Polyamid 6,12, ein Homopolykondensat aus Hexamethylendiamin und Dodecandisäure (Polyhexamethylendodecanamid) oder Polyamid 6-3-T, ein Homopolykondensat aus Trimethylhexamethylendiamin und Terephthalsäure (Polytri-methylhexamethylenterephthalamid), sowie Gemische davon. Bevorzugt sind Polycaprolactame. Bevorzugt sind uni- und insbesondere biaxial gereckte Polyamide.

Mit den Kunststoffschichten können z.B. Monofilme oder -schichten und Verbunde von zwei oder mehreren Folien, Filmen und/oder Schichten aus den genannten Kunststoffen, Kunststoffgemischen oder Misch-, Block-, Pfropf oder Copolymeren davon umfasst sein.

Die Dicke der kunststoffhaltigen Folien beträgt z.B. jeweils 12 bis 100 µm, zweckmässig 20 bis 70 µm und bevorzugt 20 bis 30 µm. Polyamidfolien und Polyethylenterephtalatfolien weisen bevorzugt eine Dicke von 20 bis 30 µm, Polyvinylchloridfolien von 30 bis 100 µm und Polyolefinfolien von 40 bis 70 µm auf.

Zur Herstellung der Laminate können die kunststoffhaltigen Folien oder Schichten und die Metallfolien gegenseitig miteinander verbunden werden. Dies kann mittels Haftvermittlern und/oder Kaschierklebern erfolgen. Die Haftung der Folien untereinander kann zudem durch Korona-, Flamm- oder Plasmavorbehandlung der Folienoberflächen gesteuert und dabei insbesondere verstärkt werden.

Bevorzugt werden als Laminate metall- und/oder kunstoffhaltigen Folien aus einer undurchlässigen Zwischenschicht und beidseitig der Zwischenschicht äusseren Schichten, wobei der Aufbau der Laminate, insbesondere bezüglich der Dicke der Schichten und der Materialien, im wesentlichen symmetrisch sein kann. Die Zwischenschicht bildet dabei die Symmetrieebene. Derartige Laminate weisen vor allem eine hohe Planlage und geringe Rolltendenz auf.

Um einen im wesentlichen symmetrischen Aufbau zu erhalten, können die Kunststoffschichten beidseits der Metallschicht des Verbundwerkstoffes beispielsweise die gleiche Dicke aufweisen oder die Dicke der beiden Kunststoffschichten differiert bevorzugt nicht mehr als 20 %, insbesondere nicht mehr als 10 %, d.h. die Dicken der Kunststoffschichten differieren bevorzugt um 0 bis 20 %, respektiv 0 bis 10 %, ihres Masses der Dicke voneinander

Die beidseitig der Metallschicht befindlichen Kunststoffschichten und insbesondere die Thermoplaste auf Polyamid-Basis können, unabhängig voneinander, jeweils ein- oder beidseitig zusätzlich mit einer aussenliegenden siegelfähigen Schicht und/oder einer Sperrschicht, z.B. aus thermoplastischen Kunststoffen, versehen sein.

Als Sperrschicht für Gase, Dämpfe, Feuchtigkeit, Geruch- und Geschmackstoffe können Folien mit Sperreigenschaften aus thermoplastischen Kunststoffen angewendet werden. Beispiele sind Folien aus Ethylenvinylalkohol-Copolymeren, Polyvinylidenchlorid, Polyacrylnitril, z.B. BAREX, Polyacryl-Polyamid-Copolymeren, Copolyester, z.B. Mitsui B-010, aromatische und amorphe Polyamiden, z.B. N-MXD6 der Mitsubishi Gas Chemical. Diese Folien können z.B. 6 µm bis 100 µm dick sein. Bevorzugt ist eine Polyvinyliden-Sperrschicht. Beispielsweise liegen Sperrschichten zwischen der Metallschicht und der oder den Schichten aus thermoplastischen Kunststoffen. Bevorzugt liegen die Sperrschichten auf den Schichten aus thermoplastischen Kunststoffen, wobei die Schichten aus thermoplastischem Kunststoffen ihrerseits auf der Metallschicht aufliegen. An der fertigen Blister- oder Durchdrückverpackung ist die Sperrschicht dann vorteilhaft gegen das Produkt oder den Inhalt gerichtet.

Der erfindungsgemässe Verbundwerkstoff kann auch auf einer oder beiden Aussenseiten eine Siegelschicht oder siegelfähige Schicht aufweisen. Beispiele dafür sind siegelbare Folien z.B. aus Polyethylenen oder Siegellacke.

Typische Beispiele von Verbundwerkstoffen für vorliegende Anwendung sind;
oPA 25/Al 45/PVC 60,
oPA 25/Al 60/PVC 60,
oPA 25/Al 45/PVC 100,
oPA 25/Al 60/PVC 100,
Al 120/PP 50,
oPA 25/Al 45/PE-beschichtet,
cPP 60/oPA 25/Al 60/cPP 60,
oPA 25/Al 60/oPA 25/EAA 50,
oPET 25/Al 45/PVC 60,
oPET 25/Al 60/oPET 25,
oPA 25/Al 45/oPVC 30 oder
vorzugsweise oPA 25/Al 60/oPA 25/HS-Lack,
wobei oPA für orientiertes (gerecktes) Polyamid, PVC für Polyvinylchlorid, oPVC für orientiertes (gerecktes) Polyvinylchlorid, PE für Polyethylen, PP für Polypropylen, cPP für gegossenes (cast) Polypropylen, EAA für Ethylenacrylsäure und Al für Aluminiumfolie steht und die Ziffern für die Schicht-, resp. Foliendicke in µm stehen. HS hat die Bedeutung von Heisssiegellack.

Schichtaufbauten für Verbundwerkstoffe mit im wesentlichen symmetrischem Aufbau nach vorliegender Erfindung enthalten beispielsweise:
a) eine mittlere Schicht aus Aluminium in einer Dicke von beispielsweise 8 bis 80 µm, bevorzugt von 40 bis 70 µm und insbesondere 45 bis 60 µm und beidseitig der Aluminiumschicht nacheinander,
b) und b') je eine Schicht eines Kaschierklebers und/oder Haftvermittlers in einer Dicke von 1,5 bis 9 µm, resp. 1 bis 10g/m^{2,}
c) und c') je eine Schicht eines biaxial gereckten Polyolefins, Polyamides oder Polyethylenterephthalates in einer Dicke von beispielsweise 12 bis 70 µm, bevorzugt 20 bis 40 µm und insbesondere 20 bis 30 µm
   und gegebenenfalls
d) und/oder d') eine Sperrschicht oder je eine Sperrschicht
   und gegebenenfalls
e) und/oder e') eine Schicht eines Siegellackes oder eine Siegelschicht oder je eine Schicht eines Siegellackes oder eine Siegelschicht in Mengen von 20 bis 6 g/m², resp. in 2 bis 10 µm Dicke.

Beispiele von Schichtfolgen sind c)/b)/a)/b')/c' oder d)/c)/b)/a)/b')/c') oder e)/d)/c)/b)/a)/b')/c') oder e)/d)/c)/b)/a)/b')/c')/ d') oder e)/d)/c)/b)/a)/b')/c')/ d')/e') usw.

Aus den erfindungsgemässen Verbundwerkstoffen hergestellte Blister- oder Durchdrückpackungen oder Teile von Verpackungen müssen z.B. sowohl bei Normaldruck, als auch im Vakuum, hitzebeständig und kältebeständig sein. Deshalb sind die einzelnen Bestandteile der Verbundwerkstoffe sowohl für sich, als auch in gegenseitigem Verbund zweckmässig hitzebeständig und kältebeständig. Diese Eigenschaften treffen insbesondere für die einzelnen Kunststoffolien, allfällige Extrudate, Coextrudate oder Laminate und die verwendeten Kleber und Haftvermittler zu.

Vorliegende Erfindung betrifft auch Blister- oder Durchdrückpackungen, deren Vertiefungen ein pharmazeutisches Produkt enthalten und das pharmazeutische Produkt ein durch Lyophilisierung einer flüssigen Portion eines pharmazeutischen Produktes in der Blister- oder Durchdruckpackung erzeugter Festkörper ist.

Vorliegende Erfindung betrifft auch ein Verfahren zur Herstellung von Blister- und Durchdrückpackungen. Nach diesem Verfahren werden die metall- und/oder kunstoffhaltigen Folien tiefgezogen, streckgezogen oder gleichzeitig tief- und streckgezogen, wobei die Vertiefungen in die Folie geformt werden und im gleichen oder einem anschliessenden Arbeitsgang die negative oder positive Prägung an wenigstens einem Boden, vorzugsweise an jedem Boden, angebracht wird. Um die Folien nicht zu hoch zu dehnen und damit eine Porenbildung, welche die Barrierewirkung der Metallschicht mindert oder zerstört, zu verursachen, wird vorteilhaft eine geringe Prägetiefe von etwa 0,01 bis 3 mm und vorzugsweise 0,1 bis 0,5 mm, eingehalten.

Die Prägung kann nach verschiedenen Verfahren an den Böden der Vertiefungen angebracht werden. Beispielsweise kann die Vorrichtung zur Formung der Bodenteile der Blister- oder Durchdrückpackungen einen Stempel, als Gegenstück eine Matrize und einen Niederhalter aufweisen. Die zu verformende metall- und/oder kunstoffhaltige Folie wird zwischen der Matrize und dem Niederhalter festgeklemmt oder die Folie wird soweit festgehalten, dass sie begrenzt nachfliessen kann. Somit können die metall- und/oder kunstoffhaltigen Folien, vorzugsweisedurch Kaltverformung, tiefgezogen, streckgezogen oder gleichzeitig tief- und streckgezogen werden. Der Stempel formt die Konturen der Vertiefung. Entsprechend der vorgesehenen Prägung können die Stempelenden, welche die Böden formen, ein negatives oder positives Prägemuster aufweisen. Es ist möglich die Vertiefungen und die Prägung in den Böden der Vertiefungen mit einem Stempel in einem oder mehreren Stempelhüben zu formen. Es können auch die Vertiefungen mit einem Stempel in einem oder mehreren Stempelhüben oder mittels einer Serie von Stempeln in mehreren Stempelhüben geformt werden. Mit einem separaten Prägestempel kann die Prägung an den Böden vor oder nach der Formung der Vertiefungen oder zwischen zwei Stempelhüben bei der Formung der Vertiefungen angebracht werden. In weiterer möglicher Ausführungsform wird die Folie mit einem Vorstreckstempel, der auch das Prägemuster aufweist, verformt und mit einem zweiten Stempel in einem zweiten Verformungsschritt die Vertiefung auf die endgültige Tiefe verformt. Der Vorstreckstempel kann eine verformungswirksame Oberfläche hoher Reibung aufweisen. Z.B. Polyacetale (POM) erfüllen diese Vorgabe. Es ist auch möglich als Stempel teleskopisch ineinander angeordnete Stempeleinsätze anzuwenden und das Prägemuster an einem oder mehreren Stempeleinsätzen anzubringen. Die Stempeleinsätze können durch gesteuertes Absenken die Vertiefungen in der Folie ausformen und die Prägungen anbringen. Anstelle eines mechanisch wirkenden Stempels kann die Verformung der Folie mittels Druckgas, wie Druckluft, oder Vakuum oder durch Hydroformen erfolgen. Es ist auch möglich nach der mechanischen Formung der Vertiefungen mittels Druckluft, Vakuum oder Hydroformen das am Stempel angebrachte Prägemuster auf die Böden der Vertiefung zu übertragen.

Der Stempel kann, zumindest im Bereich der verformungswirksamen Oberflächen, aus Kunststoffen, wie Tetrafluorethylen, Polyacetalen oder Polyethylen usw., aus Metall, wie Aluminium, Stahl, Blei usw., aus keramischen Materialien oder einer Kombination dieser Materialien aufgebaut sein.

Die Matrize stellt bezüglich der Vertiefungen im wesentlichen das gegenteilige Abbild des Stempels dar. Die Matrize kann im Bereich der Prägung aus elastisch verformbaren Material, wie Gummi, Hartgummi, Naturkautschuk, synthetischem Kautschuk, wie durch Polymerisation erhältlicher Kautschuk der Typen Polybutadien, Styrol-Butadien-Kautschuk, Acrylnitril-Butadien-kautschuk, Poly-2-chlorbutadien, Polyisopren, Äthylen-Propylen-Kautschuk, Butylkautschuk, Äthylen-Vinylacetat-Copolymere, Acrylatkautschuke, Epoxidkautschuke, Fluorkautschuke, Fluorsiliconkautschuke, Nitrosokautschuke, Polyurethankautschuke, Thioplaste, thermoplastische Elastomere (Styrol/Butadien-Blockcopolymere), durch Polykondensation und Polyaddition erhältlicher Synthesekautschuk, wie Siliconkautschuke, Synthesekautschuke erhältlich durch Umwandlung von Polymeren, wie chloriertes Polyethylen, chlorsulfoniertes Polyethylen, Chlorbutylkautschuk und Brombutylkautschuk, oder elastischen Kunststoffen oder aus anderweitig verformbaren Materialien, wie Kunststoffen, Holz oder weichem Metall, insbesondere Blei, sein. Die Shore-Härte A nach DIN 53505 der Matrize kann -- insbesondere im Bereich der Prägung -- beispielsweise von 5 bis 95, vorzugsweise von 15 bis 75 und insbesondere von 30 bis 60, betragen.

Die Matrize und dabei insbesondere verformbare Matrizen können im Bereich der verformungswirksamen Oberflächen für die Böden glatt, d.h. ohne Prägemuster, sein. Die Prägung der Folie erfolgt dann durch das am Stempel angebrachten Prägemuster, unter Verformung der Matrize im Bereich der Prägung. Die Matrize kann auch komplementär zum Stempel das negative oder positive Abbild des Prägemusters am Stempel aufweisen. Entsprechend erfolgt in diesem Falle kaum eine oder keine Verformung der Matrize.

Die prägungswirksamen Teile, d.h. im wesentlichen das Prägemuster, sowohl am Stempel als auch an der Matrize, können durch Giessen der Matrize oder des Stempels erzeugt werden. Stempel und Matrize können auch durch mechanische oder chemische Verfahren, wie durch Erosion, Funkenerosion, durch Fräsen, Drehen, oder Stanzen usw. erzeugt werden. Zum leichten und kostengünstigen Wechsel der Prägemuster können die prägungswirksamen Teile an Stempel und/oder Matrize austauschbar ausgeführt sein.

Zur Herstellung der erfindungsgemässen Blister- oder Durchdrückpackungen werden die metall- und/oder kunstoffhaltigen Folien, vorzugsweise durch Kaltverformung, zu Bodenteilen von Blister- oder Durchdrückpackungen tiefgezogen, streckgezogen oder gleichzeitig tief- und streckgezogen, wobei die Vertiefungen in die Folie geformt werden und im gleichen oder einem anschliessenden Arbeitsgang das negative oder positive Prägung an wenigstens einem Boden, vorzugsweise an jedem Boden, angebracht und ein pharmazeutisches Produkt als flüssige Portion in jede Vertiefung eingefüllt und durch Lyophilisation die Flüssigkeit entfernt wird, wobei sich das verbleibende pharmazeutische Produkt verfestigt und gleichzeitig das positive Prägung im Boden der Vertiefung als Negativform in das sich verfestigende Präparat einprägt oder das negative Prägung im Boden der Vertiefung als Positivform in das sich verfestigende Präparat einprägt.

Die Kaltverformung kann beispielsweise ab Raumtemperatur bis etwa 50°C erfolgen. Fallweise kann einer Warmverformung bei Temperaturen von über 50° C bis ca. 140°C der Vorzug gegeben werden.

Nach dem vorliegenden Verfahren werden bei der Lyophilisation oder Gefriertrocknung die Bodenteile der Blister- oder Durchdrückpackungen mit den Vertiefungen, in die das pharmazeutische Produkt als Mischung von Flüssigkeit und Feststoff, d.h. als Lösung oder Suspension, eingefüllt worden ist, unter Vakuum Temperaturen bis zu -196°C ausgesetzt. Dabei gefrieren und anschliessend verdampfen oder sublimieren die ursprünglich flüssigen Anteile und es bleiben die Feststoffe -- zumindest annähernd -- in Tablettenform, zurück. Die Bedingungen während des Lyophilisierens stellen hohe Ansprüche bezüglich der Festigkeit gegen Delamination des eingesetzten Laminates, gegen Aufwölbung oder Rolltendenz und der Masshaltigkeit der Bodenteile für die weitere maschinelle Verarbeitung, wie Bedeckelung.

Die Bedeckelung und damit Versiegelung des pharmazeutischen Produktes gegen Ausseneinflüsse, kann beispielsweise durch Aufsiegeln oder Aufkleben einer glatten, unverformten Deckelfolie, wie einer Aluminiumfolie, einer mit Kunststofffilmen laminierten Aluminiumfolie, einer ein- oder mehrschichtigen Kunststofffolie, einem mit Kunststoffen beschichteten Papier, einem Laminat aus Papier, Metallschicht und gegebenenfalls Kunststoffschichten, usw. erfolgen. Die Deckelfolie wird vorteilhaft über den Schulterbereich, welcher die Vertiefungen am Bodenteil umgibt, angesiegelt oder angeklebt. Es ist auch möglich, als Deckelfolie vorliegende metall- und/oder kunststoffhaltige Folie, mit oder ohne Vertiefungen, zur Bedeckelung einzusetzen. Weist der Deckel Vertiefungen auf, so kommen sinngemäss die Vertiefungen des Bodenteils und des Deckels übereinander zu liegen und bilden einen entsprechend grossen Hof. Bei der therapeutischen Anwendung des pharmazeutischen Produktes kann die Deckelfolie abgeschält und das pharmazeutische Produkt direkt auf die Zunge des Patienten gedrückt werden. Damit gelangen die Wirkstoffe über die Schleimhäute und/oder den Verdauungstrakt sehr schnell in die Blutbahn. Die Tabletten weisen das Abbild der Prägungen, die erfindungsgemäss an den Böden der Vertiefungen angebracht worden sind, auf. Somit ist eine Charakterisierung der Tabletten ohne einen Bedruckungs- oder Färbeprozess bei einem Tablettierverfahren möglich.

Vorliegende Erfindung betrifft auch die Verwendung der erfindungsgemässen Blister- oder Durchdrückpackungen aus metall- und/oder kunststoffhaltigen Folien als Blister oder Durchdrückpackung oder als Bodenteil einer Blister- oder Durchdrückpackung für pharmazeutische Produkte und bevorzugt als Träger während der Lyophilisierung von in Flüssigkeit aufgenommenen pharmazeutischen Produkten und gleichzeitig als Blister- oder Durchdrückpackung, resp. als Bodenteil davon, für die auf dem Träger tablettenförmig verfestigten pharmazeutischen Produkte, wobei die verfestigten Produkte ein Abbild der Prägung im Boden der Vertiefung aufweisen.

## Patentansprüche

1. Blister- oder Durchdrückpackungen, enthaltend metall- und/oder kunststoffhaltigen Folien mit darin eingeformten Vertiefungen, wobei jede Vertiefung eine Seitenwandung und einen Boden aufweist,
dadurch gekennzeichnet, dass
wenigstens der Boden einer Vertiefung eine positive oder negative Prägung aufweist.

2. Blister- oder Durchdrückpackungen nach Anspruch 1, dadurch gekennzeichnet, dass alle Böden eine positive oder negative Prägung aufweisen.

3. Blister- oder Durchdrückpackungen nach Anspruch 1, dadurch gekennzeichnet, dass die Prägung ein Schriftbild, ein Brailleschriftbild, ein Logogramm, ein Piktogramm, ein Ideogramm oder ein Relief darstellt.

4. Blister- oder Durchdrückpackungen nach Anspruch 1, dadurch gekennzeichnet, dass die Vertiefungen ein pharmazeutisches Produkt enthalten und die Böden eine positive Prägung aufweisen und das pharmazeutische Produkt auf der dem Boden zugewandten Seile das Negativ der Prägung aufweisen oder die Böden eine negative Prägung aufweisen und das pharmazeutische Produkt auf der dem Boden zugewandten Seite das Positiv der Prägung aufweist.

5. Blister- oder Durchdrückpackungen nach Anspruch 4, dadurch gekennzeichnet, dass die Vertiefungen ein pharmazeutisches Produkt enthalten und das pharmazeutische Produkt ein durch Lyophilisierung einer flüssigen Portion eines pharmazeutischen Produktes in der Blister- oder Durchdrückpackung erzeugter Festkörper ist.

6. Blister- oder Durchdrückpackungen nach Anspruch 1, dadurch gekennzeichnet, dass die metall- und/oder kunstoffhaltigen Folien Laminate sind, enthaltend eine undurchlässige Zwischenschicht und beidseitig der Zwischenschicht angeordnet äussere Schichten, wobei die äusseren Schichten gleiche thermische Ausdehnungkoeffizienten aufweisen.

7. Blister- oder Durchdrückpackungen nach Anspruch 1, dadurch gekennzeichnet, dass die metall- und/oder kunstoffhaltigen Folien Laminate sind, aus einer undurchlässigen Zwischenschicht, zweckmässig einer Metallschicht, und beidseitig der Zwischenschicht angeordnet, äussere Schichten, wobei die äusseren Schichten Kunstoffschichten sind und die Kunststoffschichten vorzugsweise Thermoplaste auf der Basis von Polyamiden oder Polyethylenterephthalaten enthalten.

8. Blister- oder Durchdrückpackungen nach Anspruch 7, dadurch gekennzeichnet, dass die Zwischenschicht eine Aluminiumschicht ist und die beidseitig der Zwischenschicht angeordneten äusseren Schichten Polyamidfolien sind.

9. Blister- oder Durchdruckpackungen nach Anspruch 1, dadurch gekennzeichnet, dass die metall- und/oder kunstoffhaltigen Folien Laminate sind, enthaltend die Schichten:
oPA 25/Al 45/PVC 60,
oPA 25/Al 60/PVC 60,
oPA 25/Al 45/PVC 100,
oPA 25/Al 60/PVC 100,
Al 120/PP 50,
oPA 25/Al 45/PE-beschichtet,
cPP 60/oPA 25/Al 60/cPP 60,
oPA 25/Al 60/oPA 25/EAA 50,
oPET 25/Al 45/PVC 60,
oPET 25/Al 60/oPET 25,
oPA 25/Al 45/oPVC 30 oder
vorzugsweise oPA 25/Al 60/oPA 25/HS-Lack,
wobei oPA für orientiertes Polyamid, PVC für Polyvinylchlorid, PE für Polyethylen, PP für Polypropylen, cPP für gegossenes (cast) Polypropylen, EAA für Ethylenacrylsäure und Al für Aluminiumfolie steht und die Ziffern für die Schicht-, resp. Foliendicke in µm stehen und HS die Bedeutung von Heisssiegellack hat.

10. Verfahren zur Herstellung von Blister- oder Durchdrückpackungen nach Anspruch 1, dadurch gekennzeichnet, dass die metall- und/oder kunstoffhaltigen Folien tiefgezogen, streckgezogen oder gleichzeitig tief- und streckgezogen werden, wobei die Vertiefungen in die Folie geformt werden und im gleichen oder einem anschliessenden Arbeitsgang die negative oder positive Prägung an wenigstens einem Boden, vorzugsweise an jedem Boden, angebracht wird.

11. Verfahren zur Herstellung von Blister- oder Durchdrückpackungen nach Anspruch 10, dadurch gekennzeichnet, dass die metall- und/oder kunstoffhaltigen Folien durch Kaltverformung tiefgezogen, streckgezogen oder gleichzeitig tief- und streckgezogen werden.

12. Verfahren zur Herstellung von Blister- oder Durchdrückpackungen nach Anspruch 10, dadurch gekennzeichnet, dass die zu verformende metall- und/oder kunstoffhaltige Folie zwischen einer Matrize und einem Niederhalter festgeklemmt wird oder die Folie soweit festgehalten wird, dass die Folie begrenzt nachfliessen kann, die Folie tiefgezogen, streckgezogen oder gleichzeitig tief- und streckgezogen wird, wobei der Stempel die Konturen der Vertiefung formt und die Stempelenden, welche die Böden formen, ein negatives oder positives Prägemuster aufweisen und der Stempel in einem oder mehreren Stempelhüben die Vertiefungen und die Prägungen formt oder mittels einer Serie von Stempeln in mehreren Stempelhüben die Vertiefungen und die Prägungen geformt werden oder mit einem separaten Prägestempel die Prägungen an den Böden vor oder nach der Formung der Vertiefungen oder zwischen zwei Stempelhüben bei der Formung der Vertiefungen angebracht werden, oder der Stempel weist teleskopisch ineinander angeordnete Stempeleinsätze auf und das Prägemuster ist an einem oder mehreren Stempeleinsätzen angebracht und die Stempeleinsätze formen durch gesteuertes Absenken die Vertiefungen in der Folie und bringen die Prägung an.

13. Verfahren zur Herstellung von Blister- oder Durchdrückpackungen nach Anspruch 12, dadurch gekennzeichnet, dass die metall- und/oder kunstoffhaltige Folie mit einem Vorstreckstempel, der auch das Prägemuster aufweist, verformt und mit einem zweiten Stempel in einem zweiten Verformungsschritt die Vertiefung auf die endgültige Tiefe verformt wird und der Vorstreckstempel eine verformungswirksame Oberfläche aus Polyacetal (POM) aufweist.

14. Verfahren zur Herstellung von Blister- oder Durchdrückpackungen nach Anspruch 10, dadurch gekennzeichnet, dass die metall- und/oder kunstoffhaltigen Folien tiefgezogen, streckgezogen oder gleichzeitig tief- und streckgezogen werden, wobei die Vertiefungen in die Folie geformt werden und im gleichen oder einem anschliessenden Arbeitsgang die negative oder positive Prägung an wenigstens einem Boden, vorzugsweise an jedem Boden, angebracht und ein pharmazeutisches Produkt als flüssige Portion in jede Vertiefung eingefüllt und durch Lyophilisation die Flüssigkeit entfernt wird, wobei sich das verbleibende pharmazeutische Produkt verfestigt und gleichzeitig die positive Prägung im Boden der Vertiefung als Negativ in das sich verfestigende Präparat einprägt oder die negative Prägung im Boden der Vertiefung als Positiv in das sich verfestigende Präparat einprägt.

15. Verfahren zur Herstellung von Blister- oder Durchdrückpackungen nach Anspruch 10, dadurch gekennzeichnet, dass die Verformung der metall- und/oder kunstoffhaltigen Folie mittels Druckgas, wie Druckluft, oder Vakuum oder durch Hydroformen erfolgt oder nach einer mechanischen Formung der Vertiefungen mit Matrize und Stempel mittels Druckgas, Vakuum oder Hydroformen das am Stempel angebrachte Prägemuster auf die Böden der Vertiefungen übertragen wird.

16. Verfahren zur Herstellung von Blister- oder Durchdrückpackungen nach Anspruch 10, dadurch gekennzeichnet, dass die Verformung der metall- und/oder kunstoffhaltigen Folie mit Matrize und Stempel erfolgt und die Matrize im Bereich der verformungswirksamen Oberflächen für die Böden glatt und verformbar ist und die Prägung der Folie durch das am Stempel angebrachten Prägemuster, unter Verformung der Matrize im Bereich der Prägung, erfolgt.

17. Verfahren zur Herstellung von Blister- oder Durchdrückpackungen nach Anspruch 16, dadurch gekennzeichnet, dass die Verformung der metall- und/oder kunstoffhaltigen Folie mit Matrize und Stemel erfolgt und die Matrize im Bereich der verformungswirksamen Oberflächen für die Böden glatt und verformbar ist und die Prägung der Folie durch das am Stempel angebrachten Prägemuster, unter Verformung der Matrize im Bereich der Prägung, erfolgt und die Matrize aus elastisch verformbaren Material mit einer Shore-Härte A nach DIN 53505 von 5 bis 95, zweckmässig von 15 bis 75 und vorzugsweise von 30 bis 60 ist.

18. Verfahren zur Herstellung von Blister- oder Durchdrückpackungen nach Anspruch 17, dadurch gekennzeichnet, dass die Verformung der metall- und/oder kunstoffhaltigen Folie mit Matrize und Stempel erfolgt und die Matrize im Bereich der verformungswirksamen Oberflächen für die Böden glatt und verformbar ist und die Prägung der Folie durch das am Stempel angebrachten Prägemuster, unter Verformung der Matrize im Bereich der Prägung, erfolgt und die Matrize im Bereich der Prägung aus elastisch verformbaren Material, der Reihe Gummi, Hartgummi, Naturkautschuk, synthetischem Kautschuk, durch Polymerisation erhältlicher Kautschuk der Typen Polybutadien, Styrol-Butadien-Kautschuk, Acrylnitril-Butadien-kautschuk, Poly-2-chlorbutadien, Polyisopren, Äthylen-Propylen-Kautschuk, Butylkautschuk, Äthylen-Vinylacetat-Copolymere, Acrylatkautschuke, Epoxid-Kautschuke, Fluorkautschuke, durch Polykondensation und Polyaddition erhältlicher Synthesekautschuk, Siliconkautschuke, Synthesekautschuke erhältlich durch Umwandlung von Polymeren, chloriertes Polyethylen, chlorsulfoniertes Polyethylen, Chlorbutylkautschuk und Brombutylkautschuk, Nitrosokautschuk, Polyurethankautschuk, Thioplaste, thermoplastische Elastomere oder elastische Kunststoffe oder aus verformbaren Materialien, Kunststoffen, Holz oder weichem Metall ist.

19. Verwendung der Blister- oder Durchdrückpackung aus metall- und/oder kunststoffhaltigen Folien nach Anspruch 1 als Blister oder Durchdrückpackung oder als Bodenteil einer Blister- oder Durchdrückpackung für pharmazeutische Produkte und bevorzugt als Träger während der Lyophilisierung von in Flüssigkeit aufgenommenen pharmazeutischen Produkten und gleichzeitig als Blister- oder Durchdrückpackung oder als Bodenteil der Blister- oder Durchdrückpackung, für die tablettenförmig verfestigten pharmazeutischen Produkte, wobei die verfestigten Produkte ein Abbild der Prägung im Boden der Vertiefung aufweisen.
